# EUROPEAN PATENT APPLICATION

(11) **EP 0 557 642 A2**
(43) Date of publication of application: **01.09.1993**
(21) Application number: 92309412.2
(22) Date of filing: 15.10.1992
(51) Int. Cl.: G01N 33/02, G01N 33/14, G01N 27/416

(54) **Method of measuring diacetyl in processed drinks and foods**

(30) Priority: 27.02.1992 JP 78696/92
(71) Applicant: KAGOME KABUSHIKI KAISHA, Nagoya-shi Aichi-ken (JP)
(72) Inventor: Horikawa, Naoyuki, Tokyo-to (JP); Hayakawa, Kiro, Nasu-gun, Tochigi-ken (JP); Yamada, Yasunori, Nishinasuno-cho Nasu-gun, Tochigi-ken (JP)
(74) Representative: Ablewhite, Alan James

(57) **Abstract**

Diacetyl content in a processed drink or food material is easily and quickly measured by adding a buffer solution to a fixed amount thereof to adjust its pH value, say, to 3-7, subjecting to a deoxygenation process, thereafter adding o-phenylenediamine to cause a reaction, say, at 30-70°C to obtain a reaction liquid and analyzing this reaction liquid by differential pulse voltammetry.

## Description

This invention relates to a method of measuring the amount of diacetyl contained in processed drinks and foods.

Many kinds of drinks and foods are based on agricultural, marine and live stock products. Diacetyl is frequently contained in such processed drinks and foods, although to various degrees, seriously affecting their quality, and the flavor in particular. For example, although diacetyl is a flavor-providing component in fermented dairy products such as cheeses and yogurt, it adversely affects the quality of processed drinks such as tomato juice and apple juice. In either situation, therefore, it is important to know the content of diacetyl at the final stage of the production process or during the production of such processed drinks and foods if it is desired to provide high quality products. The present invention relates to a method of measuring diacetyl contained in such processed drinks and foods.

Gas and liquid chromatography methods have been used conventionally for the measurement of diacetyl contained in processed drinks and foods. (J. of Food Quality, Vol. 8, pp 199-207, 1985; Bunseki Kagaku, Vol. 39, pp 405-409, 1990). Although these methods have the advantage of providing high accuracy, they are disadvantageous in that they require very troublesome operations, taking a long time for the measurement. In order to produce high quality products by obtaining data on the contained amount of diacetyl at the final stage of or during the production process of processed drinks and foods, not only should the accuracy of measurement be high, but also the operation for the measurement must be simple and the measurement itself should not be too time-consuming.

It is therefore an object of the present invention to provide a simply executable, yet accurate method of measuring diacetyl contained in processed drinks and foods.

It is another object of the present invention to provide such a method which does not take a long time to carry out.

A method embodying the present invention, with which the above and other objects can be accomplished, may be characterized as comprising the steps of adding a buffer solution to a fixed amount of processed drink or food product, collected as a sample, to thereby adjust its pH value, carrying out a deoxygenation process, thereafter adding o-phenylenediamine and using its reaction product with diacetyl in the buffered drink or food product to carry out a differential pulse voltammentry process.

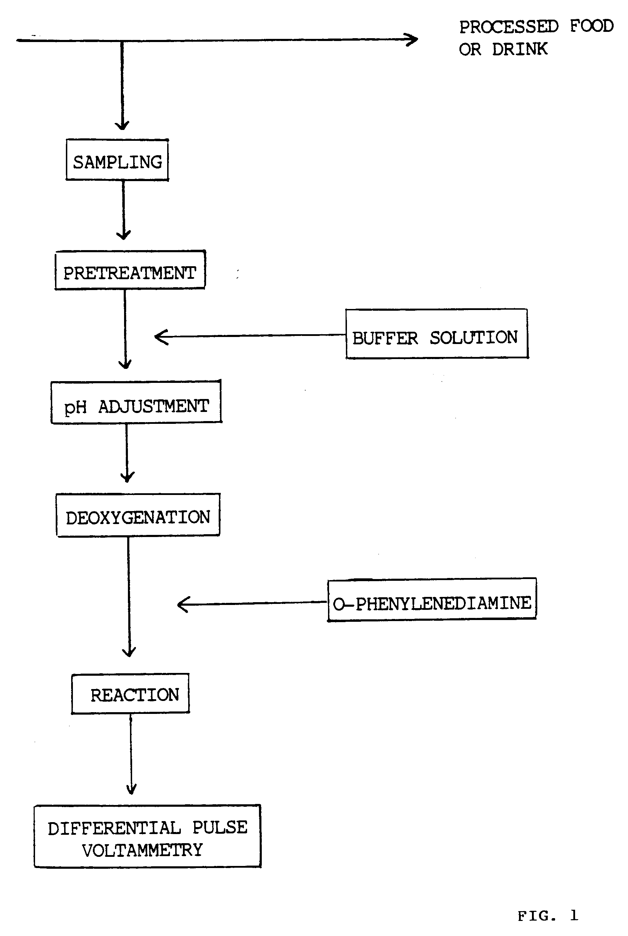

Fig. 1, which is incorporated in and forms a part of this specification, is a flow chart of a method embodying the present invention and, together with the description, serves to explain the principles of the invention.

According to the present invention, a fixed amount of processed drink or food is collected, and this collected sample is subjected to a deoxygenation process after a buffer solution is added thereto for adjustment of its pH value. Depending on the nature and/or conditions of the collected sample, it may be preferable to subject it to a pretreatment process, such as filtering, centrifugal separation or distillation, either before or after the adjustment of the pH value. In the case of a processed drink containing a large amount of pulp such as tomato juice, for example, it is removed by filtering or centrifugal separation.
Alternatively, the sample may be distilled not only to remove the pulp component but also to condense the sample. In the case of a sample such as yogurt that contains a large amount of protein, on the other hand, protein may be removed by ultrafiltration.

The buffer solution to be used for the adjustment of pH value must have superior buffering ability. The pH value is normally adjusted in the acid range. The range of pH 3-7 is desirable, the range of pH 5-7 being further preferable. This is for the purpose of stabilizing the reaction between diacetyl contained in the processed drink or food and o-phenylenediamine, which is to be added later, such that the measurement of diacetyl can be accomplished more accurately and in a shorter period of time.

In the deoxygenation process, nitrogen gas, for example, is passed through the pH-adjusted solution in order to remove the oxygen contained dissolved in the solution. This is because such dissolved oxygen would adversely affect the sensitivity when o-phenylenediamine is later added for a reaction and its reaction product is used in a differential pulse voltammetry process.

According to the present invention, o-phenylenediamine is added to this deoxygenation solution for a reaction. This is for the purpose of causing the diacetyl contained in this solution to react with o-phenylenediamine and to thereby become transformed into 2,3-dimethylquinoxaline. Only a suitable amount of o-phenylenediamine for transforming diacetyl into 2,3-dimethylquinoxaline is required, but it is preferred to add about 5-50 times, and more preferably about 5-10 times, the amount of diacetyl. If an excessively large amount of o-phenylenediamine is added, an electric current peak corresponding to o-phenylenediamine will appear when the reaction product is later used in differential pulse voltammetry.

The reaction between diacetyl and o-phenylenediamine may be carried out at any temperature which is generally allowed, but a temperature range of about 30-70°C, and in particular of about 50-60°C, is preferred in order to complete the reaction even more quickly.

According to the present invention, the reaction liquid obtained by the addition of o-phenylenediamine is used in a differential pulse voltammetry process. Differential pulse voltammetry is similar to D.C. polarography, which is well known. While a linearly increasing pulse voltage is applied in pulse voltammetry, differential pulse voltammetry provides a method of superposing a constant pulse voltage at a regular time interval onto a D.C. voltage which increases at a constant rate, and has been used for the measurement of heavy metals such as copper, lead and cadmium. For the differential pulse voltammetry, it is preferable to use a glassy carbon electrode as its working electrode for the purpose of improving the sensitivity for 2,3-dimethylquinoxaiine in the reaction liquid.

With the reaction liquid thus used in differential Pulse voltammetry, a peak current value is read and compared with preliminarily prepared reference lines to thereby determine the concentration of diacetyl in the tested sample. Such reference lines are prepared by adding a commercially available standard diacetyl reagent to the same processed drink or food at several different levels of concentration, processing these samples similarly to obtain their respective current peak values and plotting against diacetyl concentration the differences between these peak values and the peak value corresponding to a blank sample. The reference lines thus obtained are straight.

The steps of the procedure described above are summarized in the flow chart of Fig. 1. From the production process of a processed drink or food product, a sample is collected and, after a pretreatment, a buffer solution is added to have its pH value adjusted. The pH-adjusted liquid is deoxygenated and o-phenylenediamine is added and reacted, the reaction liquid being used in a differential pulse voltammetry process. By such a method according to the present invention, diacetyl concentrations up to 0.08ppm can be detected with good repeatability in less than 1/4 of the time required by prior art methods with gas or liquid chromatography.

In order to more clearly demonstrate the effects of the present invention, results of experiments using both methods embodying the present invention and conventional methods will be described in what follows. The Examples should not be considered limiting.

### Example 1

In Example 1, a sample of 200ml was taken from the final stage of production process for tomato juice, and an initial distillate of 4ml was obtained by distilling this sample. To this was added 96ml of a buffer solution (Britton-Robinson buffer, 0.04M acetate + 0.04M phosphate + 0.04M boronate) and the pH value was adjusted to 5.0. Next, nitrogen gas was passed through this pH-adjusted liquid for 10 minutes to remove the oxygen remaining dissolved. Thereafter, o-phenylenediamine was added to this deoxygenated solution such that its concentration became 5ppm and was caused to react therewith for 15 minutes at 50°C. The reaction liquid thus obtained was used in a differential pulse voltammetry process under the following conditions:
Apparatus: Yanako polarographic analyzer P-1100 (Trade name of Kabushiki Kaisha Yanagimoto Seisakusho);
Working electrode: Glassy carbon with electrode area of 0.237cm²;
Reference electrode: Silver/Silver chloride;
Sweep potential: 0 - 1.0V;
Scan rate: 10mV/second;
Modulation amplitude: 50mV;
Pulse interval: 0.5 seconds.

The electric current peak value was read and compared with preliminarily prepared reference lines. The diacetyl concentration in the tomato juice was determined to be 0.09ppm.

### Comparison Example 1

In Comparison Example 1, 200ml of tomato juice was taken as in Example 1, and 100ml of distilled water and 50ml of ether were added to it for steam distillation in order to separate an ether layer. This ether layer was stored for 12 hours at -20°C and after the moisture was removed by freezing, it was condensed to 0.1ml by means of an evaporator. This condensed liquid was analyzed by gas chromatography under the following conditions:
Apparatus: Gas chromatography apparatus produced by Packard Co., Ltd. (with temperature controller and temperature programmer);
Detector: FID;
Column: Porapak Q (Trade name of Waters Associates Co., Ltd. with glass column of 6 feet x 1/4 inch OD);
Injection temperature: 210°C;
Detector temperature: 200°C;
Column temperature: 100-200°C (10°C/minute);
Carrier gas: Nitrogen gas;
Flow rate: 45ml/minute;
Injection volumn: 2µl.

The result was compared with reference lines preliminarily prepared in a similar manner as explained above. The measured diacetyl concentration in the tomato juice was 0.09ppm.

### Example 2

In Example 2, 80ml of the same buffer solution as used in Example 1 was added to 20g of commercially available yogurt so as to adjust its pH value to 5.0. Protein was removed from this pH-adjusted liquid by ultrafiltration with a filter membrane having separation molecular weight of 20,000. Nitrogen gas was passed through this filtered liquid for 10 minutes to remove oxygen remaining dissolved inside:
o-phenylenediamine was then added to this deoxygenated solution such that its concentration would become 5ppm and the reaction was continued for 20 minutes at 40°C. The reaction liquid thus obtained was used in a differential pulse voltammetry process under the same conditions as in Example 1. The electric current peak value was read and compared with preliminarily prepared reference lines. The diacetyl concentration in the yogurt sample was determined to be

### Comparison Example 2

In Comparison Example 2, 50ml of methyl alcohol was added to 10g of the same yogurt as used in Example 2. After they were fully mixed, a clear liquid was obtained in an upper layer by centrifugal separation at 2000rpm for 5 minutes. Next, 1.0ml of 2,4-dinitrophenylhydrazine (a derivative reagent for HPLC by GL Sciences Co., Ltd.) was added to 1ml of this clear liquid and they were mixed with the container tightly sealed. After they were reacted for 60 minutes at 27°C, a filtrate was obtained by using a 0.45µm filter. The filtrate thus obtained was analyzed by liquid chromatography under the following conditions:
Apparatus: Tritorter-V (Trade name) of Nihon Bunko Kabushiki Kaisha;
Detector: Spectrophotometer (365nm);
Column: Reverse phase chromatography TSKgel ODS-80Tm (Trade name: 250mm x 4.6mm) of Tosoh Kabushiki Kaisha;
Column temperature: 50°C;
Mobile phase: Acetonitrile/Water = 50/50 (V/V);
Flow rate: 1.0ml/minute;
Injection volume: 20µl.

The result was compared with reference lines preliminarily prepared in a similar manner as explained above. The measured diacetyl concentration in the yogurt sample was 2.0ppm.

It should be clear from the above that the present invention provides a method which can be easily executed for measuring diacetyl contained in processed drinks and foods in a much shorter time than by conventional methods and can hence be used even in the production process for processed drinks and foods without substantially interrupting the production process.

## Claims

1. A method of measuring the concentration of diacetyl contained in a processed drink or food material, said method comprising the steps of:
collecting a fixed amount of said material;
adding a buffer solution to said fixed amount of material to adjust its pH value to thereby obtain a pH-adjusted solution;
carrying out a deoxygenation process on said pH-adjusted solution to produce a deoxygenated solution;
thereafter adding o-phenylenediamine to said deoxygenated solution to cause a reaction and to thereby obtain a reaction liquid; and
analyzing said reaction liquid by a differential pulse voltammetry process.

2. The method of Claim 1 further comprising the step of subjecting said fixed amount of material to a pretreatment process prior to said deoxygenation process, said pretreatment process being selected from the group consisting of filtering, centrifugal separation and distillation.

3. The method of Claim 1 or Claim 2 wherein the pH value is adjusted to 3-7.

4. The method of any of Claims 1 to 3 wherein 5-50 times as much o-phenylenediamine is added as the anticipated amount of diacetyl in said fixed amount of material.

5. The method of any of Claims 1 to 4 wherein said reaction is carried out at 30-70°C.

6. The method of any of Claims 1 to 5 wherein said differential pulse voltammetry process is carried out by using a glassy carbon electrode as a working electrode.
